# NEW EUROPEAN PATENT SPECIFICATION

(11) **EP 0 584 068 B2**
(45) Date of publication and mention of the opposition decision: **28.04.2004**
(45) Mention of the grant of the patent: 06.10.1999
(21) Application number: 91912579.9
(22) Date of filing: 16.05.1991
(51) Int. Cl.: A61K 31/52

(54) **METHOD AND COMPOSITION FOR AMELIORATING THE ADVERSE EFFECTS OF AGING**
VERFAHREN UND ZUSAMMENSETZUNG ZUR VERBESSERUNG DER UNGÜNSTIGEN AUSWIRKUNGEN DES ALTERNS
PROCEDE ET COMPOSITION PERMETTANT D'AMELIORER LES EFFETS FACHEUX DU VIEILLISSEMENT

(43) Date of publication of application: 02.03.1994
(73) Proprietor: SENETEK, PLC, St. Louis, MO 63141 (US)
(72) Inventor: RATTAN, Suresh, I., S., DK-8210 Aarhus V (DK)
(74) Representative: Allard, Susan Joyce
(86) International application number: PCT/US1991/003466
(87) International publication number: WO 1992/020341

(56) References cited:
- FR-A- 1 587 633
- US-A- 4 853 386
- US-A- 5 021 422
- DATABASE WPI Week 8511, Derwent Publications Ltd., London, GB; AN 85-064790 & JP-A-60 019 709 (YOKOYAMA, N.) 31 January 1985
- The Merck Index, 9th ed. pub. 1976, page 696, paragraph 5159.

## Description

### TECHNICAL FIELD

This invention is directed to methods and compositions for ameliorating the adverse effects of aging in mammalian cells without increasing the cells' growth rate or total proliferative capacity.

More specifically, the invention relates to methods of use of known compounds, kinetin and other 6-(substituted amino) purine cytokinins, for countering the adverse effects of aging on mammalian cells in culture and in vivo, including cells of human skin, and compositions for carrying out such methods.

### BACKGROUND OF THE INVENTION

Preventing the cellular aging process has been a persistent, though elusive, goal of biological science. Preventing the aging process would have a number of significant, practical consequences. For example, the production of valuable products by cells in culture could be improved if the cells retain characteristics of young cells. Also, preventing the aging of cells in human skin or other organs would be of significant value because of the associated preservation of structural and functional integrity. In the case of skin, ameliorating the adverse effects of cellular aging would also advantageously involve preservation of cosmetic integrity.

A number of attributes have been identified to differentiate between young and aged cells. For example, it is found that young mammalian, including human fibroblast, cells in standard culture appear healthy and clean; possess a regular, long, thin spindle-shaped morphology; are tightly packed in arrays on becoming confluent on culture substrata; do not overgrow one another; seldom have other than one nucleus; and produce little debris in the culture medium. On the other hand, fibroblast cells that are old display many age-related symptoms, such as flattened and irregular morphology, abnormally large size, sparse growth, low cell yield per unit area of culture substratum, a significant frequency of polynucleated cells, difficulty in trypsinization, and a high rate of production of debris in the culture medium. The morphological characteristics which distinguish young from old cells, as well as the high level of autofluorescence found in old cells, reflect various physiological and biochemical characteristics which distinguish young from aged cells, including high responsiveness to growth factors and higher rates of DNA and protein synthesis in young cells.

Morphological, physiological and biochemical characteristics to distinguish young from old cells have been identified largely from studies of mammalian, including human, cells (usually fibroblasts) in culture. In these studies, "age" of cells in a culture is measured by the number of doublings of cell number that have occurred from the primary culture (made from tissue) to the culture of interest derived or descended from the primary culture.

Yet, characteristics distinguishing young from old cells found in studies of cells in culture are also pertinent to cells which occur in vivo in living mammals and which share many characteristics of cultured fibroblasts, such as similar biochemical characteristics and tight control of growth (i.e., tight proliferative control). Fibroblasts of the dermal layer (i.e., dermis) of skin and the connective tissue layer underlying the epithelia of the inner wall of the gastrointestinal tract are examples of cells which display characteristics of aging similar to those found for fibroblasts in culture.

A measure of the "age" of such fibroblasts in tissues of a living mammal, which normally divide periodically under tight growth control, is the number of cell divisions which have occurred between the cells and their predecessors at about the time of birth of the mammal. Because cell division occurs periodically, there is typically a correlation between chronological age of the mammal and the age of particular cells measured by cell divisions between the cells and their predecessors at birth.

Another characteristic of fibroblasts in culture is the "total proliferative capacity" of the cells. This is measured by the total number of doublings of cell number that the culture can undergo before growth of the culture ceases, due to death (cessation of growth and division) of the "aged" cells therein. For human fibroblast cultures, this total protiferative capacity is typically about 45-60 doublings for a primary culture. As understood in the art, the cessation of culture growth appears very quickly, with growth rate (measured by reciprocal of doubling time) decreasing from near normal values characteristic of young cells to zero in only a few doublings. In cultures of normal fibroblasts, i.e., wherein the cells have not been transformed to an immortalized or "cancerous" state, "total proliferative capacity" decreases as a function of the number of doublings which separates a culture from the primary culture from which it was derived. Other terms related to the "total proliferative capacity" of normal cells in culture are the "life expectancy" and "normal life span" of the cells. The "life expectancy" or "normal life span" of normal cells is the "total proliferative capacity" of the primary culture from which the culture in which the cells occur was derived.

The fact that normal fibroblasts, particularly of human origin, have a narrowly defined (within a few doublings) total proliferative capacity in culture is a manifestation of the tight proliferative control that such cells are under. Sometimes cells which normally have a limited total proliferative capacity can be transformed to lose this limitation. Cultures of such cells can sometimes be passaged repeatedly without any apparent limit on number of passages; such cells are said to be immortalized. Immortalization is a characteristic of cancer cells. Cancer cells are under little or no proliferative control.

Measurement of total proliferative capacity for cells in vivo in living mammals, such as fibroblasts of the dermis of the skin or the connective tissue layer underlying the intestinal epithelium, cannot be simply measured, as for fibroblasts in culture, in terms of a fixed number of doublings, limited to a narrow range characteristic of the species of the mammal (e.g., 45-60 for human, as indicated above; about 10 for murine). However, it is clear that such cells in living mammals, if the cells are normal, have a limited capacity to divide and are under tight proliferative control.

For example, fibroblasts in primary cultures established with normal cells from human adult dermis are known to have lower total proliferative capacity than fibroblasts in primary cultures established with normal cells from human fetal or newborn dermis. Indeed, from studies with large number of human donors, it is apparent that fibroblasts in primary cultures established with normal dermal-layer cells have total proliferative capacities in culture that are inversely related to donor age.

The loss of limited total proliferative capacity characteristic of cells in culture, that are transformed to the immortalized or "cancerous" state, is often accompanied by an abnormally increased rate of proliferation, referred to herein as "growth rate." Rate of proliferation, or growth rate, is a measure of the rate at which cells divide.

Similarly, the loss of tight proliferative control, that characterizes cancer cells in vivo, is often accompanied by an abnormally increased growth rate of the cells.

In prior attempts to preserve the "young" phenotype of aging cells in vivo, the growth rates of the cells have invariably been increased. It has been found, however, that increasing the growth rate of cells, which necessarily increases the rate of cell division, increases the risk that the cells become transformed to the immortalized or cancerous state.

Similarly, treatments which artificially increase the total proliferative capacity of cells in culture beyond its normal limit have been found to increase the risk of transformation to the immortalized or cancerous state.

The risks of causing transformation by tampering with the tight proliferative control on cells, by tampering with growth rates or limits on total proliferative capacity or both, are presumably due to effects of such tampering on the cells' genomes or biochemical mechanisms for controlling gene expression.

Further, treatments that, at least with cells in culture, increase growth rate or total proliferative capacity, such as epidermal and platelet-derived growth factors, insulin, glucocorticoids, extracts of Panax ginseng, and gibberellin plant hormones, are generally effective in preserving the "young" phenotype of only "young" cells, for which treatments are needed least. "Old" cells, those with at least about 80-90% of their normal life span over, respond little, if at all, to treatment with even high concentrations of these substances (much higher than those effective with "young" cells with less than about half of their life spans over).

Any composition which is to be employed to ameliorate the adverse effects of aging on cells, and which increases growth rate or total proliferative capacity of cells, in vivo or in culture, must be viewed with caution, at least to the extent the composition is to be employed on tissues (i.e., skin, intestinal wall) which include cells normally under tight proliferative control.

Prior to the present invention, compositions have not been available to ameliorate the adverse effects of aging on morphological, physiological, and biochemical characteristics of cells without potentially harmful increases in the cells' growth rate or total proliferative capacity.

It is pertinent to the present invention to understand the role of fibroblasts, particularly in the dermis off human skin but also in the corresponding connective tissue layer underlying the integuments of other mammals and the epithelia of the inner wall of the gastrointestinal tracts of humans and other animals. The fibroblasts synthesize a number of components required for maintenance of the structural, functional and cosmetic integrity of the skin and the structural and functional integrity of these other surface tissues covered by epithelia. These components include collagen and elastin, which are fibrous proteins responsible for the three-dimensional architecture of skin and the other surface tissues; fibronectin, a protein which is responsible for cell anchorage and maintenance of cell morphology; and a number of proteinaceous growth factors essential for the maintenance of epithelia and basal cell layers and connective tissue layers underlying them. Because of the important role of protein synthesis by fibroblasts in maintaining the health of skin and other surface tissues and because available evidence indicates that protein biosynthetic activity of fibroblasts decreases significantly with age (e.g., rate of collagen synthesis, at about 70% of life expectancy for fetal-derived human fibroblasts in culture, only about 50% of that of such fibroblasts at less than 20% of life expectancy), methods and compositions to ameliorate the adverse effects of aging on protein biosynthetic activity of fibroblasts in vivo, without affecting the total proliferative capacity or growth rate of such fibroblasts, would be especially advantageous.

The present invention involves 6-(substituted amino) purine cytokinins, which include kinetin and 6-amino purine analogs thereof, of Formula I: 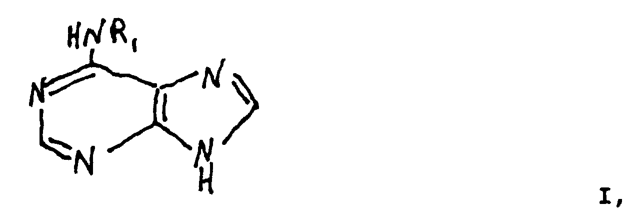 wherein R₁ is furfuryl, phenyl, benzyl, n-alkyl of 4, 5 or 6 carbons, (cyclohexyl)methyl, 3,3-dimethylallyl, or 3-hydroxymethyl-3-methylallyl.

The cytokinins are a class of plant hormones defined by their ability to promote cell division in plant tissue explants in standard media which contain auxins (another class of plant hormones) as well as vitamins, mineral salts, and sugar.

Ribonucleotides in which a cytokinin, particularly kinetin (6-furfuryl-aminopurine), zeatin (6-(3-hydroxymethyl-3-methylallyl)-aminopurine), or 6-(3,3-dimethylallyl)-aminopurine, is the base moiety have been identified as components of some transfer RNAs in certain plant and animal cells.

Kinetin is known to form complexes with certain RNA-binding proteins of wheat embryo extracts and has been suggested to promote protein synthesis in plants. Spirin and Ajtkhozhin, Trends in Biochem. Sci., April, 1985, p. 162.

Furthermore, kinetin has been used in the production of protein-rich yeast employing conventional culture methods wherein kinetin is added to the culture medium. East German Patent No. 148,889 (1981) (Derwent World Patent Index Abstract). Kinetin has also been reported to augment the growth of microbial cultures. Merck Index, 10th Ed., Entry 5148 (Merck and Co., Rahway, New Jersey, U.S.A., 1983).

Finally, Japanese Patent No. 60019709 (1985) discloses a composition which comprises no more than 1% of kinetin and no more than 20% of an uncharacterized lithospermum root extract which is said to accelerate cell division in human skin and thereby prevent skin-aging.

### SUMMARY OF THE INVENTION

It has now been discovered, surprisingly, that exposing mammalian cells to various 6-(substituted amino) purine cytokinins ameliorates the adverse effects of aging on such cells by delaying the onset of, and even reversing, the age-related morphological changes that normally occur with aging of the cells, and that such exposure has this ameliorative effect without increasing the growth rate or total proliferative capacity of the cells.

Based on this discovery, novel compositions and methods are provided for ameliorating, including reversing, the adverse effects of aging on mammalian cells, including such cells in culture and in vivo, such as in human skin.

Thus, among other applications, the methods and compositions of the invention are useful in slowing and reversing the degeneration of the structural, functional and cosmetic integrity of human skin that normally accompanies aging.

As discussed above, all compositions heretofore available to ameliorate the adverse effects of aging on biochemical, physiological or morphological characteristics of mammalian cells also undesirably increase the growth rate or total proliferative capacity of the cells.

### DETAILED DESCRIPTION OF THE INVENTION

In one aspect, the present invention entails a method of ameliorating the adverse effects of aging on mammalian cells, which method comprises administering to said cells a composition which comprises, at a concentration which is effective to ameliorate said adverse effects on such cells, a cytokinin, which is a 6-(substituted amino)purine.

In another aspect, the invention entails a composition for ameliorating the adverse effects of aging on mammalian cells, said composition comprising, at a concentration which is effective to ameliorate said adverse effects on such cells, a cytokinin, which is a 6-(substituted amino) purine.

Among the 6-(substituted amino)purine cytokinins which can be employed as the adverse-age-effect-ameliorating-effective compound in the methods and compositions of the invention are kinetin, zeatin, 6-(3,3-dimethylallyl)aminopurine, 6-(benzyl)aminopurine, 6-(phenyl)aminopurine, 6-(n-alkyl)aminopurine, wherein the n-alkyl group as 4, 5 or 6 carbons, and 6-(cyclohexyl)methylaminopurine. Most preferred is kinetin (6-(furfuryl)aminopurine).

The mammalian cells, which can be treated with the methods and compositions of the invention, include human cells, and cells in culture as well as cells in vivo in living mammals.

The cells, if in culture, can be derived from any tissue and, if in vivo, can be part of any tissue. The methods and compositions are preferably applied with fibroblasts in tissues in vivo in humans, such as fibroblasts of the dermis of human skin or the connective tissue layer underlying the epithelium of the inner wall of the human small intestine or stomach. The most preferred application is to cells in human skin.

Thus, the present invention further involves methods, and compositions for carrying them out, for slowing (or reversing) the degeneration, which normally accompanies aging, of the structural and functional integrity of skin and epithelia of the inner wall of the gastrointestinal tract and the cosmetic integrity of skin.

The methods and compositions of the invention are effective at surprisingly and advantageously low concentrations of the 6-(substituted amino)purine cytokinin. The concentrations are typically between 10⁻⁶ M and 5 x 10⁻⁴ M in a tissue culture medium. At these concentrations, the cytokinin apparently has no toxic effect on mammalian cells.

Surprisingly, and especially advantageously, the methods of the invention do not significantly affect either the growth rate or the total proliferative capacity of cells treated according to the methods. The compositions and methods of the present invention do not affect the rate or amount of DNA synthesis in treated cells. Unlike other compositions for preserving or restoring the "young" phenotype of aging cells, the compositions and methods of the present invention do not affect the tight proliferative control under which many cells, in vivo, such as fibroblasts and certain cells of the epidermis and other epithelia, must remain to function normally.

By "ameliorating the adverse effects of aging" on mammalian cells is meant that the development of the morphological changes, described earlier, that normally occur with "aging" in normal mammalian cells in culture or in vivo is slowed or reversed.

The skilled can ascertain whether a composition, to which mammalian cells are exposed in carrying out the method of the invention, includes an amount of a 6-(substituted amino)purine cytokinin that is effective to ameliorate the adverse effects of aging on the cells. Morphological changes associated with aging are easily discerned by the skilled by microscopic examination of cells, removed from a culture, taken by biopsy from tissue, or the like.

In the case of mammalian cells in culture, which may be grown directly from tissue without genetic engineering or may be genetically modified to produce a desired product by any of various known genetic engineering techniques, and which, as understood in the art, can be used to make valuable proteins, such as lymphokines or hormones, for diagnostic, therapeutic or other applications, ameliorating the adverse effects of aging with the methods and compositions of the present invention advantageously maintains the cells "younger" than the cells actually are. Indeed, addition of an effective amount of a 6-(substituted amino) purine cytokinin to a culture medium of mammalian cells, that have been passaged numerous times and that have morphological characteristics characteristic of aging cells, reverses the morphological characteristics to those of younger cells.

The advantages provided by compositions and methods of the present invention in preserving, and, in the case of reversing adverse effects of aging, improving the functional integrity of mammalian cells in vivo, such as fibroblasts of the dermis of the skin or the connective tissue layer underlying the epithelia of the inner wall of the gastrointestinal tract of humans, and, in particular, in preserving the cosmetic integrity of human skin, are apparent and have been discussed above.

Compositions according to the invention can take any of a number of forms, depending on the nature and the surroundings of the cells to which adverse-age-effect-ameliorative amounts of 6-(substituted amino)purine cytokinins are to be applied in accordance with the invention.

For cells in culture, bathed or suspended in a culture medium, a composition according to the invention is any culture medium used for mammalian cells which is supplemented with between about 10⁻⁶ M (i.e., 1 µM) and about 5 x 10⁻⁴ M of a 6-(substituted amino)purine cytokinin. For the preferred cytokinins, of Formula I 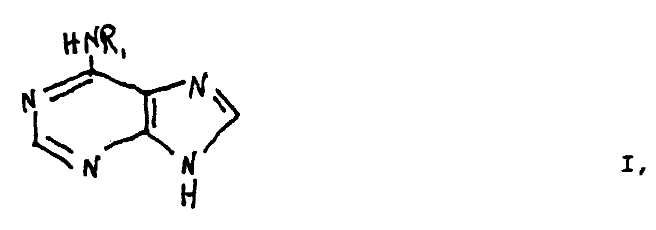 wherein R₁ is furfuryl, phenyl, benzyl, n-alkyl of 4, 5 or 6 carbons, (cyclohexyl)methyl(-CH₂(C₆H₁₁)), 3-hydroxymethyl-3-methylallyl 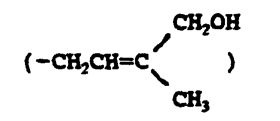 or 3,3-dimethylallyl (-CH₂CH=C(CH₃)₂), the concentration range in the culture medium is between 0.2 and 100 parts per million (ppm). For the most preferred cytokinin, kinetin, the preferred concentration range is about 25 µM to about 250 µM (i.e., about 5 ppm to about 50 ppm) in the medium.

In the case of the cells in culture, the method of the invention of administering to the cells an adverse-age-effect-ameliorative amount of a 6-(substituted amino)purine cytokinin, is carried out by simply bathing or growing the cells in a culture medium which is a composition according to the invention.

The method of the invention is carried out with cells in vivo in mammals, preferably fibroblasts (or other active cells, e.g., keratinocytes) of the dermis of human skin, or the connective tissue layer underlying the epithelia of the inner wall of the human gastrointestinal tract, by exposing such cells to an adverse-age-effect-ameliorative amount of a 6-(substituted amino)purine cytokinin.

Generally such exposure will be repeated periodically over the period of time during which it is desired to ameliorate the adverse age effects on the cells in accordance with the invention. The frequency of exposure will depend on the cells involved, where in the mammal the cells are located (e.g., cells in the gastrointestinal tract, where flushing repeatedly by compositions free of 6-(substituted amino)purine cytokinins occurs, requiring more frequent exposure than cells of the skin), and the concentration of the 6-(substituted amino)purine cytokinin in a concentration range, typically between about 10 ppm and about 1000 ppm), effective to permeate to the connective tissue layer (underlying the epithelia of the inner wall of the gastrointestinal tract) at a concentration effective to ameliorate the adverse effects of aging on active cells, such as fibroblasts, in said connective tissue layer.

For the connective tissue layer underlying the epithelia of the inner wall of the gastrointestinal tract, the method of the invention is carried out by ingestion of a composition according to the invention which is a solution, suspension, or tonic that is physiologically acceptable for oral consumption and which comprises a 6-(substituted amino) purine cytokinin in a concentration range, typically between about 10 ppm and about 1000 ppm, effective to permeate the connective tissue layer underlying the epithelia of the inner wall of the gastrointestinal tract to a concentration effective to ameliorate the adverse effects of aging on active cells, such as fibroblasts, in said connective tissue layer.

For fibroblasts (or other active cells, such as keratinocytes) of the human skin, the method of the invention is carried out by applying to the outer surface of the skin a composition according to the invention, which is a cream, ointment, lotion, gel, solution, perfume, solid, or the like, which is physiologically acceptable for application to the outer surface of the skin and which comprises a 6-(substituted amino)purine cytokinin in a suitable vehicle at an effective concentration, i.e., a concentration effective to permeate to the dermis of the skin at a concentration effective to ameliorate the adverse effects of aging on active cells (e.g., fibroblasts) in said dermis.

Compositions of the invention for use with human skin are formulated with a 6-(substituted amino)purine cytokinin, preferably one of those of above-defined Formula I, in an effective concentration range, typically between about 0.5 ppm and about 500 ppm in a cream, ointment, lotion, gel, solution or solid base or vehicle known in the art to be non-toxic and dermatologically acceptable. The concentration (or weight fraction) of 6-(substituted amino)purine cytokinin dissolved, suspended, dispersed, or the like in a composition of the invention for use with human skin will be such that the 6-(substituted amino)purine cytokinin is delivered at between about 0.2 ppm and about 100 ppm to active cells of the skin, such as fibroblasts. Generally, emollient or lubricating vehicles which help hydrate the skin are more preferred than volatile vehicles, such as ethanol, which dry the skin.

Examples of suitable bases or vehicles for preparing compositions of the invention for use with human skin are petrolatum, petrolatum plus volatile silicones, lanolin, cold cream (USP), and hydrophilic ointment (USP). A preferred composition of the invention has between about 10 ppm and 100 ppm of kinetin in combination with a suitable ointment or cream base.

The choice of an acceptable vehicle is largely determined by the way the 6-(substituted amino)purine cytokinin is to be administered. Such methods include topical administration. Suitable pharmaceutically and dermatologically acceptable vehicles for topical application include those suited for use in lotions, creams, solutions, gels, tapes and the like. Generally, the vehicle is either organic in nature or an aqueous emulsion and capable of having the 6-(substituted amino)purine cytokinin dispersed, suspended or dissolved therein. The vehicle may include pharmaceutically-acceptable emollients, skin penetration enhancers, coloring agents, fragrances, emulsifiers, thickening agents, and solvents. A more detailed description of such forms follows:

### 1. Lotions

The lotions can comprise an effective amount of a 6-(substituted amino)purine cytokinin (e.g., an amount effective to deliver the 6-(substituted amino)purine cytokinin at between about 0.2 ppm and about 100 ppm to active cells of the skin, such as fibroblasts); from 1% to 50%, preferably from 3% to 15%, of an emollient; the balance being water, a C₂ or C₃ alcohol, or a mixture of water and the alcohol. Several emollients are known. Examples of such emollients are as follows:
a. Hydrocarbon oils and waxes. Examples are mineral oil, petrolatum, paraffin, ceresin, ozokerite, microcrystalline wax, polyethylene, and perhydrosqualene.
b. Silicone oils, such as dimethylpolysiloxanes, methylphenylpolysiloxanes, water-soluble and alcohol-soluble silicone-glycol copolymers.
c. Triglyceride fats and oils such as those derived from vegetable, animal and marine sources. Examples include (but are not limited to) castor oil, safflower oil, cotton seed oil, corn oil, olive oil, cod liver oil, almond oil, avocado oil, palm oil, sesame oil, and soybean oil.
d. Acetoglyceride esters, such as acetylated monoglycerides.
e. Ethoxylated glycerides, such as ethoxylated glyceryl monstearate.
f. Alkyl esters of fatty acids having 10 to 20 carbon atoms. Methyl, isopropyl and butyl esters of fatty acids are useful herein. Examples include (but are not limited to) hexyl laurate, isohexyl laurate, isohexyl palmitate, isopropyl palmitate, isopropyl myristate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
g. Alkenyl esters of fatty acids having 10 to 20 carbon atoms. Examples thereof include (but are not limited to) oleyl myristate, oleyl stearate, and oleyl oleate.
h. Fatty acids having 9 to 22 carbon atoms. Suitable examples include (but are not limited to) pelargonic, lauric, myristic, palmitic, stearic, isostearic, hydroxystearic, oleic, linoleic, ricinoleic, arachidonic, behenic, and erucic acids.
i. Fatty alcohols having 10 to 22 carbon atoms. Lauryl, myristyl, cetyl, hexadecyl, stearyl, isostearyl, hydroxystearyl, oleyl, ricinoleyl, behenyl, erucyl, and 2-octyl dodecyl alcohols are examples of satisfactory fatty alcohols.
j. Fatty alcohol ethers. Ethoxylated fatty alcohols of 10 to 20 carbon atoms include (but are not limited to) the lauryl, cetyl, stearyl, isostearyl, oleyl, and cholesterol alcohols having attached thereto from 1 to 50 ethylene oxide groups or 1 to 50 propylene oxide groups, or a mixture thereof.
k. Ether-esters, such as fatty acid esters of ethoxylated fatty alcohols.
l. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases are illustrative of emollients derived from lanolin.
m. Polyhydric alcohols and polyether derivatives. Propylene glycol, dipropylene glycol, polypropylene glycol (M.W. 2000-4000), polyoxyethylene polyoxypropylene glycols, polyoxypropylene polyoxyethylene glycols, glycerol, ethoxylated glycerol, propoxylated glycerol, sorbitol, ethoxylated sorbitol, hydroxypropyl sorbitol, polyethylene glycol (M.W. 200-6000), methoxy polyethylene glycols 350, 550, 750, 2000, 5000, poly[ethylene oxide] homopolymers (M.W. 100,000-5,000,000), polyalkylene glycols and derivatives, hexylene glycol (2-methyl-2,4-pentanediol), 1,3-butylene glycol, 1,2,6-hexanetriol, ethohexadiol USP (2-ethyl-1,3-hexanediol), C₁₅-C₁₈ vicinal glycol, and polyoxypropylene derivatives of trimethylolpropane are examples thereof.
n. Polyhydric alcohol esters. Ethylene glycol mono- and di-fatty acid esters, diethylene glycol mono- and di-fatty acid esters, polyethylene glycol (M.W. 200-6000), mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
o. Wax esters, such as beeswax, spermaceti, myristyl myristate, and stearyl stearate.
p. Beeswax derivatives, e.g., polyoxyethylene sorbitol beeswax. These are reaction products of beeswax with ethoxylated sorbitol of varying ethylene oxide content, forming a mixture of ether-esters.
q. Vegetable waxes, including (but not limited to) carnauba and candelilla waxes.
r. Phospholipids, such as lecithin and derivatives.
s. Sterols. Cholesterol and cholesterol fatty acid esters are examples thereof.
t. Amides, such as fatty acid amides, ethoxylated fatty acid amides, and solid fatty acid alkanolamides.

The lotions further preferably comprise from 1% to 10%, more preferably from 2% to 5%, of an emulsifier. The emulsifiers can be nonionic, anionic or cationic. Examples of satisfactory nonionic emulsifiers include (but are not limited to) fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono-and di-fatty acid esters of ethylene oxide, mono- and di-fatty acid esters of ethylene glycol wherein the fatty acid moiety contains from 10 to 20 carbon atoms, diethylene glycol, polyethylene glycols of molecular weight 200 to 6000, propylene glycols of molecular weight 200 to 3000, glycerol, sorbitol, sorbitan, polyoxyethylene sorbitol, polyoxyethylene sorbitan and hydrophilic wax esters. Suitable anionic emulsifiers include (but are not limited to) the fatty acid soaps, e.g. sodium, potassium and triethanolamine soaps, wherein the fatty acid moiety contains from 10 to 20 carbon atoms. Other suitable anionic emulsifiers include (but are not limited to) the alkali metal, ammonium or substituted ammonium alkyl sulfates, alkyl arylsulfonates, and alkyl ethoxy ether sulfonates having 10 to 30 carbon atoms in the alkyl moiety. The alkyl ethoxy ether sulfonates contain from 1 to 50 ethylene oxide units. Among satisfactory cationic emulsifiers are quaternary ammonium, morpholinium and pyridinium compounds. Certain of the emollients described in preceding paragraphs also have emulsifying properties. When a lotion is formulated containing such an emollient, an additional emulsifier is not needed, though it can be included in the composition.

The balance of the lotion is water or a C₂ or C₃ alcohol, or a mixture of water and the alcohol. The lotions are formulated by simply admixing all of the components together. Preferably the 6-(substituted amino)purine cytokinin is dissolved in the mixture.

Conventional optional components can be included. One such additive is a thickening agent at a level from 1% to 10% of the composition. Examples of suitable thickening agents include (but are not limited to): cross-linked carboxypolymethylene polymers, ethyl cellulose, polyethylene glycols, gum tragacanth, gum kharaya, xanthan gums and bentonite, hydroxyethyl cellulose, and hydroxypropyl cellulose.

### 2. Creams

The creams comprise an effective amount of a 6-(substituted amino)purine cytokinin (e.g., an amount effective to deliver the 6-(substituted amino)purine cytokinin at between about 0.2 ppm and about 100 ppm to active cells of the skin, such as fibroblasts); from 5% to 50%, preferably from 10% to 25%, of an emollient, the balance being water. The emollients above described can also be used in the cream compositions. Optionally the cream form contains a suitable emulsifier, as previously described. When an emulsifier is included, it is in the composition at a level from 3% to 50%, preferably from 5% to 20%.

### 3. Solutions

The solution form comprises an effective amount of a 6-(substituted amino)purine cytokinin (e.g., an amount effective to deliver the 6-(substituted amino)purine cytokinin at between about 0.2 ppm and about 100 ppm to active cells of the skin, such as fibroblasts); the balance being water, a suitable organic solvent, or a combination of water and such an organic solvent. Suitable organic materials useful as the solvent or a part of a solvent system are as follows: propylene glycol, polyethylene glycol (M.W. 200-600), polypropylene glycol (M.W. 425-2025), glycerine, sorbitol esters, 1,2,6-hexanetriol, ethanol, isopropanol, diethyl tartrate, butanediol, and mixtures thereof. Such solvent systems can also contain water.

These compositions in solution form can be applied to the skin as is, or else can be formulated into an aerosol and applied to the skin as a spray-on. The aerosol compositions further comprise from 25% to 80%, preferably from 30% to 50%, of a suitable propellant. Examples of such propellants are the chlorinated, fluorinated and chlorofluorinated lower molecular weight hydrocarbons. Nitrous oxide, carbon dioxide, butane, and propane are also used as propellant gases. These propellants are used as understood in the art in a quantity and under a pressure suitable to expel the contents of the container.

### 4. Gels

Gel compositions can be formulated by simply admixing a suitable thickening agent to the previously described solution compositions. Examples of suitable thickening agents have been previously described with respect to the lotions.

The gelled compositions comprise an effective amount of a 6-(substituted amino)purine cytokinin (e.g., an amount effective to deliver the 6-(substituted amino)purine cytokinin at between about 0.2 ppm and about 100 ppm to active cells of the skin, such as fibroblasts); from 5% to 75%. preferably from 10% to 50%, of an organic solvent as previously described; from 0.5% to 20%, preferably from 1% to 10% of the thickening agent; the balance being water.

### 5. Solids

Compositions of solid forms have use as stick-type compositions intended for application to the lips or other parts of the body. Such compositions comprise an effective amount of a 6-(substituted amino)purine cytokinin (e.g., an amount effective to deliver the 6-(substituted amino)purine cytokinin at between about 0.2 ppm and about 100 ppm to active cells of the skin, such as fibroblasts), and from 50% to 98%, preferably from 60% to 90%, of the previously described emollients. This composition can further comprise from 1% to 20%, preferably from 5% to 15%, of a suitable thickening agent, and optionally emulsifiers and water. Thickening agents previously described with respect to lotions are suitably employed in the compositions in solid form.

An example of a composition of the invention suitable for application to human facial skin would consist of 10 ppm to 100 ppm of kinetin in a base prepared by mixing (by weight) 10 parts glycerol monostearate, 10 parts cetyl alcohol, 30 parts spermaceti, 10 parts Tween 20 (polyoxyalkylene derivative off sorbitan monostearate), 10 parts Span 20 (sorbitan monolaurate), 12.5 parts glycerin, and 100 parts water. Examples of adverse-age-effect-ameliorative active ingredients which could be used in place off kinetin are zeatin, 6-(3,3-dimethylallyl) aminopurine, 6-benzylaminopurine, or a combination of kinetin and 6-(n-hexyl)aminopurine or 6-benzylaminopurine.

Other ingredients, such as preservatives (e.g., methyl-paraben or ethyl-paraben), perfumes, dyes or the like, which are known in the art to provide desirable stability, fragrance or color, or other desirable properties, such as shielding from actinic rays from the sun, to compositions for application to the skin may also be employed in a composition of the invention for such topical application.

A composition of the invention may also include adverse-age-effect-ameliorative active ingredients other than 6-(substituted amino)purine cytokinins. However, one advantage of employing such cytokinins alone, that the growth rate and total proliferative capacity of treated cells are not significantly affected, will tend to be diluted if other such ingredients are employed which increase growth rate or total proliferative capacity, or both, of treated cells. Consequently, preferred compositions of the invention will comprise, as the only adverse-age-effect-ameliorative ingredient, a 6-(substituted amino)purine cytokinin.

Compositions of the invention for use with human skin will preferably be applied once per day to the areas of the skin which are desired to be treated by the method of the invention.

The method of the invention will be applied to a person's skin, by applying thereto, preferably daily, a composition of the invention suitable for human skin treatment, as discussed above, for an indefinite period, i.e., as long as the person desires to enjoy the amelioration of the adverse effects of aging of the skin provided by the method and composition of the invention. Once daily application to the skin of a composition according to the invention will be required for at least about a month, and up to at least about a year, depending on the age of the person, the condition of the skin to which the composition is applied, and the concentration (or weight fraction) of the 6-(substituted amino)purine cytokinin in the composition, before the beneficial effects, of delaying decrease in protein synthesis rate and delaying age-related morphological changes in fibroblasts of the basal cell layer of the skin, begin to become apparent at the skin surface. If application of the method of the invention is terminated, the aging effects ameliorated by the method will again ensue after some time.

The invention will now be illustrated in the following examples.

### EXAMPLE I

### EFFECT OF KINETIN ON SHORT-TERM CELL GROWTH

Effects of kinetin on short-term growth of normal diploid human embryonic skin fibroblasts, strain designated as KIS, was studied by what is known in the art as "one-step growth curve" experiments. For this purpose, equal numbers of KIS cells (10⁵) were added to several culture flasks containing Dulbecco's modified Eagle's medium (DMEM) supplemented with 10% fetal calf serum, antibiotics and glutamine. Different volumes of stock solutions of kinetin prepared in balanced salt solution (Hank's Buffer) were added to the flasks in order to obtain final concentrations of 40 µM, 80 µM and 200 pM kinetin in the culture medium. Cultures were then incubated at 37°C in an atmosphere of 5% CO₂. All experiments were done in triplicate.

In order to determine the attachment frequency of cells treated with various doses of kinetin, one set of culture flasks from each dose was used to count the number off cells attached to the bottom surface off the culture flask 6 hours after seeding. This was done by first removing the cells by trypsinization, resuspending them in fresh medium and counting their number using a Coulter counter.

For the estimation of growth rates or proliferation rates off kinetin-treated and untreated cells, cell number was determined every 24 hours in each set as above. This was continued until all cultures because confluent and there was no further increase in cell number (usually 8-10 days). The following results were obtained.
1. Normal human cells display no adverse morphological effects on treatment with various doses of kinetin as judged by microscopic examination of the cells under a light microscope.
2. Kinetin had no apparent toxic effects on human cells, because there was no difference in cell attachment, cellular debris in the medium and cell number per unit area of culture substratum between treated and untreated cells.
3. Both kinetin-treated and untreated human cells demonstrated the same growth rate and became confluent at the same time. This is clear evidence that kinetin did not increase the growth rates of cells in culture. Further evidence in support of this is presented in Example II.

### EXAMPLE II

### EFFECT OF KINETIN ON LONG-TEAM GROWTH RATE AND TOTAL PROLIFERATIVE CAPACITY

Effects of kinetin on longer term growth rate and total proliferative capacity of KIS cells were studied by what is known in the art as "longevity curve" experiments. For this purpose, at least sixteen parallel cultures of KIS cells were maintained in the presence of 40 µM kinetin in the medium, along with an equal number off untreated control cultures. All experiments were started from population doubling level (PDL) six, which is about 12% off the normal life expectancy (i.e., total proliferative capacity of the cells being about PDL = 50) in vitro as determined previously.

Methods off cell culture, trypsinization and counting the cell numbers were as described in Example I. For long term studies, however, cultures were serially passaged every time they became confluent. This serial passaging off cultures was continued until all cultures stopped becoming confluent despite weekly changes of culture media for 4-5 weeks. In order to estimate the exact numbers of PDL achieved by kinetin-treated and untreated cultures, cells in at least four confluent culture flasks out of 16 parallel running cultures were subdivided in a split ratio of 1:4, when young, and 1:2, when their growth rate started to slow down due to aging. The number of PDs a culture had undergone between seeding the cells and becoming confluent was determined by: log N/log 2, where N=number of cells in a confluent culture divided by the number of cells seeded originally in that flask. Finally, cumulative PDL for each culture was calculated at the end of their life span in vitro.

In terms of cumulative PDL there was no difference in the life span of human cells grown with or without a continuous presence of kinetin in the culture medium. Under this series off experiments, both types of cultures attained a maximum PDL in the range of 47 to 49. This means that the total proliferative capacity of the cells was not significantly changed by the presence of kinetin in the culture medium.

In terms of chronological time, however, kinetin-treated cells appeared to live longer (217 days) than the controls (196 days). This is because, at the end of their life span (i.e., when confluence could no longer be achieved), kinetin-treated cells continued to appear much healthier for at least two weeks and without shedding significant numbers of dead cells into the medium.

As to growth rates, slopes of the curves of numbers off population doublings as a function off time for both kinetin-treated and untreated cells were experimentally indistinguishable. Thus, the growth rates of kinetin-treated and untreated, control, cultures were not significantly different. Kinetin did not significantly alter the genetically determined, inherent limit to normal life span (i.e., total proliferative capacity) of human cells in culture.

### DNA Synthesis and Growth Rate

Cytokinins, including kinetin, are known to help complete the final stages of cell division, i.e., cytoldnesis, in plants. This suggested that cytokinins might affect DNA synthesis in mammalian cells in a way that would increase the risk that such cells, in the presence of cytokinins, would be transformed to the cancerous state.

Studies were, therefore, undertaken to see effects of kinetin on DNA synthesis of human cells in culture, using the methods of nuclear track autoradiography and ³H-thymidine-uptake in acid-insoluble material.

For autoradiography, KIS cells were grown on sterilized glass cover slips either in the presence of difference concentrations of kinetin (40 µM to 200 µM) or in its absence. After different durations of treatment (³H)thymidine (1 microcurie/ml) was added to the cultures either for a short time (2 hours) in order to determine the number of cycling cells at that time, or for longer periods (24-48 hours), to estimate the number of cells in a culture which are capable of entering S phase of the cell cycle. After the completion of the labeling period, cells were processed for autoradiography according to standard methods. At least 500 cells were counted, using a microscope, in each slide after exposure, developing and staining procedures were completed. Percentage of cycling cells (which had taken up radioactive thymidine and incorporated it into their newly synthesized DNA, producing dark nuclei autoradiographically) and non-cycling cells were thus determined. Kinetin-treated and untreated cells were found, after labeling 2 hours and longer (24-48 hours), to have experimentally indistinguishable percentages of cycling and non-cycling cells.

Similarly, estimates of ³H-thymidine uptake by kinetin-treated and untreated KIS cells, after labeling for either 90 minutes or 72 hours, in terms off dpm from acid-insoluble material per 10⁶ cells measured with a scintillation counter, showed no stimulation of DNA synthesis by treatment with kinetin.

As indicated in Example I, a large number of many independent experiments has shown that kinetin does not induce any additional proliferation of cells in human cell cultures. The experiments of this Example show that kinetin does not stimulate DNA synthesis or push cells into new cell cycles faster than control cells enter such cycles. Thus, kinetin doe not alter cellular growth rates of human cells.

### EXAMPLE III

### EFFECT OF KINETIN ON CELLULAR MORPHOLOGY

Age-related changes in the morphology of cells during serial passaging is a well-known phenomenon to cell biologists involved with studies of aging. Young human fibroblasts in culture are thin, long, spindle-shaped and are tightly packed in arrays on becoming confluent. Old cells, which have completed more than 90% of life span, are very large in size, very much flattened, irregularly shaped, much vacuolated and contain numerous so-called "residual bodies" which show intense autofluorescence on excitation with ultra-violet rays when observed under a fluorescence microscope.

KIS cells grown in the presence of 40 µM kinetin and grown without kinetin were compared morphologically at PDL = 44. The kinetin-treated cells did not have a typical morphology of old cells. Kinetin-treated cells were not significantly larger in size than young (e.g., PDL = 14) cells, while the untreated cells were. The kinetin-treated cells maintained a spindle-shaped appearance to a large extent; they were not irregularly placed; and they did not contain as many vacuoles and residual bodies as compared to those without kinetin treatment. This dramatic retention of somewhat youthful appearance, even during the terminal phases of life, is significant evidence of the ability of kinetin, at concentrations on the order of 10⁻⁶ M to 10⁻⁴ M, to delay the onset of age-related symptoms.

In another set of experiments, it was found that cells which had already become old (e.g., PDL greater than 40) could revert morphologically to a somewhat more youthful appearance on addition of kinetin to various concentrations to the culture medium. The degree of reversion depends on the extremity of old age already attained prior to exposure to kinetin, with younger cells reverting further, and on the concentration of kinetin added to the medium and the length of time the kinetin is present. For example, KIS cells with more than 80% life span completed (greater than 40 PDL) began to appear like PDL = 30 - 35 cells after 2-3 weeks with 200 µM kinetin in the culture medium.

The rate of re-acquisition of "old" morphology after kinetin is removed from the medium remains uncertain, but experiments indicate it is much lower than the rate of reversion to younger morphology after kinetin is introduced into the medium.

### Cell Yield and Size

One parameter of cellular aging in culture is the decline in cell yield (i.e., number of cells in a confluent layer) when cultures approach old age. This is primarily due to the fact that cells progressively become larger with age. Therefore, if the area of culture flasks remains the same, the number of cells per flask decreases when cells approach the end off their life span with periodic passaging. One set of cultures of KIS cells was cultured with periodic passaging without kinetin in the media. Another set off cultures of KIS cells was treated the same way except 40 µM off kinetin was included in the media. With the untreated cells, cell yield remained nearly constant at about 1.5 x 10⁶ per 25 cm² until PDL = 43 and then decreased to about 3.5 x 10⁵ per 25 cm² at PDL = 48, where growth ceased. With treated cells, cell yield remained nearly constant at about 1.5 x 10⁶ per 25 cm² until PDL = 48 and then decreased to about 7 x 10⁵ per 25 cm² at PDL = 49, where growth ceased. This maintenance off the cell yield characteristic of "young" cells, even during the last phase off life, with cells treated with kinetin is highly significant in terms of delaying the onset of some off the symptoms of aging in human cells by kinetin treatment.

## Claims

1. A method for ameliorating the adverse effects of aging on mammalian cells in culture, comprising contacting mammalian cells with a composition that contains an effective concentration of a 6-(substituted amino) purine cytokinin, wherein: the 6-(substituted amino) purine cytokinin has formula I: 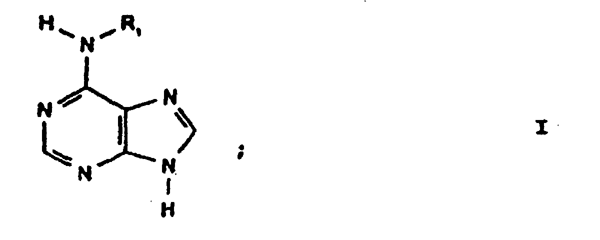 R₁ is selected from furfuryl, phenyl, benzyl, n-alkyl of 4, 5 or 6 carbons, (cyclohexyl)methyl(-CH₂(C₆H₁₁), or 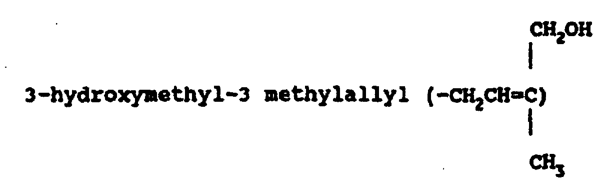 which cytokinin is the only active agent present in a concentration sufficient to ameliorate the adverse effects of aging of the cells, whereby the rate of development of characteristics of the cells that are associated with cellular aging is reversed or slowed; and the growth rate and total proliferative capacity of the cells subsequent to the contacting is substantially the same as prior to the contacting.

2. A method as claimed in claim 1 wherein the cells are fibroblast cells.

3. A method as claimed in claim 1 or claim 2 wherein the composition is culture medium.

4. A method as claimed in any of claims 1 to 3 wherein the concentration of the 6-(substituted amino) purine cytokinin is between 0.5 ppm and 500 ppm.

5. Use of a composition that contains concentration range of between 0.5 ppm to 500 ppm of a 6- (substituted amino) purine cytokinin, wherein: the 6-(substituted amino) purine cytokinin has formula I: 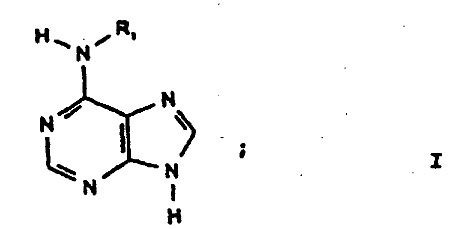 R₁ is selected from furfuryl, phenyl, benzyl, n-alkyl of 4, 5 or 6 carbons, (cyclohexyl)methyl(-CH₂(C₆H₁₁), or 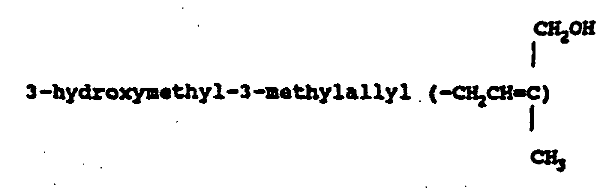 the concentration range being sufficient to ameliorate the adverse effects of aging of the cells, whereby the rate off development of characteristics of the cells that are associated with cellular aging is reversed or slowed; and the growth rate an total proliferative capacity of the cells subsequent to the contacting is substantially the same as prior to the contacting, in the manufacture of a medicament for ameliorating the adverse effects of aging an normal diplaid mammalian skin cells.

6. Use as claimed in claim 5 wherein the cells are skin fibroblasts.

7. Use as claimed in claim 6 wherein the composition is formulated as a lotion, ointment, cream, solid stick or spray for application to the surface of the skin.

8. A use or method as claimed in any one of the preceding claims wherein R₁ of formula I is furfuryl or 3-hydroxymethyl-3-methylallyl.

9. A use or method as claimed in any preceding claim wherein the concentration of the 6-(substituted amino) purine cytokinin is between 10 ppm and 100 ppm.

10. A use or method as claimed in any one of the preceding claims wherein the 6-(substituted amino) purine cytokinin is kinetin.

11. A composition for ameliorating the adverse effects of aging on mammalian cells, comprising between 0.5 ppm and 500 ppm of a 6-(substituted amino) purine cytokinin having the formula I: 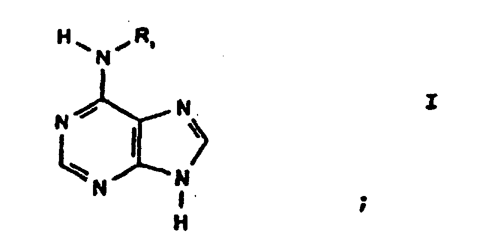 and R₁ is selected from furfuryl, phenyl, benzyl, n-alkyl of 4, 5 or 6 carbons, (cyclohexyl)methyl(-CH₂(C₆H₁₁), or

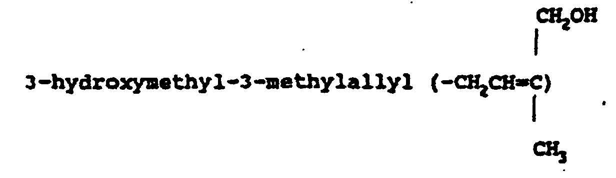

12. A composition as claimed in claim 11 which is a cosmetic.

13. A composition as claimed in claim 12 which is formulated for topical application to the surface of mammalian skin.

14. A cosmetic composition as claimed in claim 12 or claim 13 wherein the composition is formulated as a lotion, ointment, cream, solid stick or spray.

15. A composition as daimed in any one of claims 11 to 14 wherein R₁ of formula I is furfuryl or 3-hydroxymethyl-3-methylallyl.

16. A composition as claimed in any one of claims 11 to 14 wherein the 6-(substituted amino) purine cytokinin off formula I is kinetin.

17. A composition as claimed in any one of claims 11 to 16 wherein the concentration of the compound of formula I is between 10 ppm and 100 ppm.

18. An oral composition for ameliorating the adverse effects of aging on mammalian cells, comprising an effective concentration of a 6-(substituted amino) purine cytokinin in a physiologically acceptable carrier, wherein:
the 6-(substituted amino) purine cytokinin has formula I: 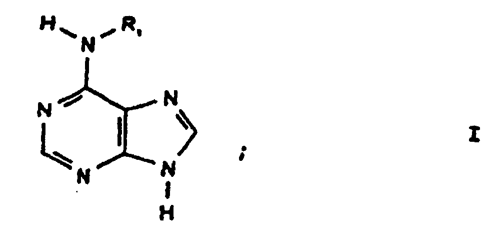
R₁ is selected from furfurly, phenyl, benzyl, n-alkyl of 4, 5 or 6 carbons, (cyclohexyl)methyl(-CH₂(C₆H₁₁), or 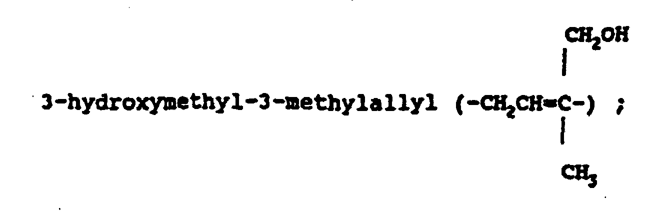 and the concentration is between about 10 ppm and about 1000 ppm and is sufficient to permeate the connective tissue layer of the gastrointestinal tract and thereby ameliorate the adverse effects of aging on the cells.

## Patentansprüche

1. Verfahren zur Verringerung von nachteiligen Alterungseffekten auf Säugerzellen in Kultur, umfassend Inkontaktbringen von Säugerzellen mit einer Zusammensetzung, welche eine wirksame Konzentration eines 6-(aminosubstituierten)-Purin-Cytokinins enthält, worin: das 6-(aminosubstituierte)-Purin-Cytokinin die Formel I aufweist: 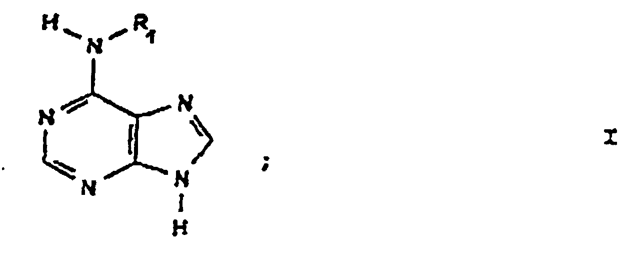 worin R₁ ausgewählt ist aus Furfuryl, Phenyl, Benzyl, n-Alkyl mit 4, 5 oder 6 Kohlenstoffatomen, (Cyclohexyl)methyl(-CH₂(C₆H₁₁), oder 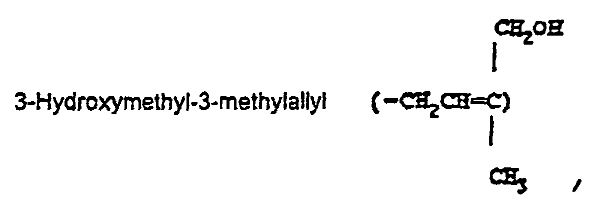 wobei das Cytokinin die einzige wirksame Substanz ist, welche in einer ausreichenden Konzentration vorhanden ist, um die nachteiligen Alterungseffekte auf die Zellen zu verringern, wobei die Entwicklungsrate von Zellmerkmalen, welche mit Zellalterung assoziiert sind, umgekehrt oder verlangsamt wird; und die Wachstumsrate und Gesamtproliferationskapazität der Zellen nach dem Inkontaktbringen im wesentlichen dieselbe ist wie vor dem Inkontaktbringen.

2. Verfahren nach Anspruch 1, worin die Zellen Fibroblastenzellen sind.

3. Verfahren nach Anspruch 1 oder 2, worin die Zusammensetzung Kulturmedium ist.

4. Verfahren nach einem der Ansprüche 1 bis 3, worin die Konzentration des 6-(aminosubstituierten)-Purin-Cytokinins zwischen 0,5 ppm und 500 ppm beträgt.

5. Verwendung einer Zusammensetzung zur Herstellung eines Arzneimittels zur Verbesserung der nachteiligen Wirkungen der Alterung auf normale diploide Säuger-Hautzellen,
welche Zusammensetzung ein 6-(aminosubstituiertes)-Purin-Cytokinin in einem Konzentrationsbereich von zwischen 0,5 ppm und 500 ppm enthält, worin das 6-(aminosubstituiertes)-Purin-Cytokinin die Formel I aufweist: 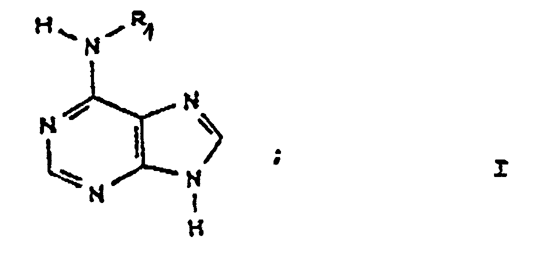 worin R₁ ausgewählt ist aus Furfuryl, Phenyl, Benzyl, n-Alkyl mit 4, 5 oder 6 Kohlenstoffatomen, (Cyclohexyl)methyl(-CH₂(C₆H₁₁), oder 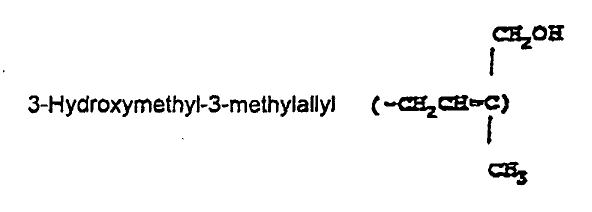 wobei der Konzentrationsbereich ausreichend ist, um die nachteiligen Alterungseffekte auf die Zellen zu verringern, wobei die Entwicklungsrate von Zellmerkmalen, die mit Zellalterung assoziiert sind, umgekehrt oder verlangsamt wird; und die Wachstumsrate und Gesamtproliferationskapazität der Zellen nach dem Inkontaktbringen im wesentlichen dieselbe ist wie vor dem Inkontaktbringen.

6. Verwendung nach Anspruch 5, worin die Zellen Hautfibroblasten sind.

7. Verwendung nach Anspruch 6, worin die Zusammensetzung als Lotion, Salbe, Creme, fester Stift oder Spray für die Anwendung auf die Obefläche der Haut formuliert wird.

8. Verwendung oder Varfahren nach einem der vorhergehenden Ansprüche, worin R₁ der Formel I Furfuryl oder 3-Hydroxymethyl-3-methylallyl ist.

9. Verwendung oder Verfahren nach einem der vorhergehenden Ansprüche, worin die Konzentration des 6-(aminosubstituiertes)-Purin-Cytokinin zwischen 10 ppm und 100 ppm beträgt.

10. Verwendung oder Verfahren nach einem der vorhergehenden Ansprüche, worin das 6-(aminosubstituierte)-Purin-Cytokinin Kinetin ist.

11. Zusammensetzung zur Verringerung von nachteiligen Alterungseffekten auf Säugerzellen, umfassend zwischen 0,5 ppm und 500 ppm eines 6-(aminosubstituierten)-Purin-Cytokinins mit der Formel I: 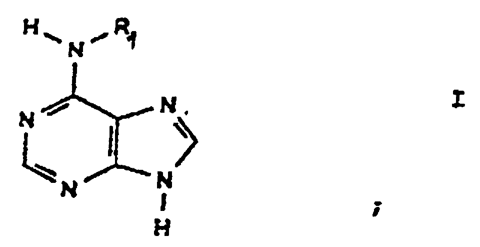 und worin R₁ ausgewählt ist aus Furfuryl, Phenyl, Benzyl, n-Alkyl mit 4, 5 oder 6 Kohlenstoffatomen, (Cyclohexyl)methyl(-CH₂(C₆H₁₁), oder

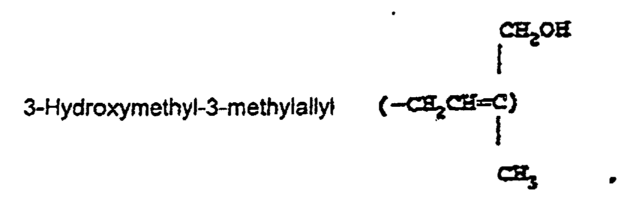

12. Zusammensetzung nach Anspruch 11, wobei die Zusammensetzung ein Kosmetikum ist.

13. Zusammensetzung nach Anspruch 12, wobei die Zusammensetzung für die topische Anwendung auf die Oberfläche von Säugerhaut formuliert ist.

14. Kosmetische Zusammensetzung nach Anspruch 12 oder 13, worin die Zusammensetzung als Lotion, Salbe, Creme, fester Stift oder Spray formuliert ist.

15. Zusammensetzung nach einem der Ansprüche 11 bis 14, worin R₁ der Formel I Furfuryl oder 3-Hydroxymethyl-3-methyallyl ist.

16. Zusammensetzung nach einem der Ansprüche 11 bis 14, worin das 6-(aminosubstituierte)-Purin-Cytokinin der Formel I Kinetin ist.

17. Zusammensetzung nach einem der Ansprüche 11 bis 16, worin die Konzentration der Verbindung der Formel I zwischen 10 ppm und 100 ppm beträgt.

18. Orale Zusammensetzung zur Verringerung von nachteiligen Alterungseffekten auf Säugerzellen, umfassend eine wirksame Konzentration eines 6-(aminosubstituierten)-Purin-Cytokinins in einem physiologisch annehmbaren Träger, worin das 6-(aminosubstituierte)-Purin-Cytokinin die Formel I aufweist: 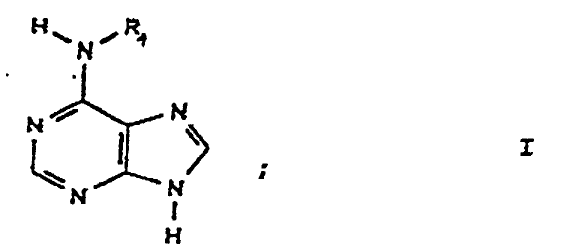 worin R₁ ausgewählt ist aus Furfuryl, Phenyl, Benzyl, n-Alkyl mit 4, 5 oder 6 Kohlenstoffatomen, (Cyclohexyl)methyl(-CH₂(C₆H₁₁), oder 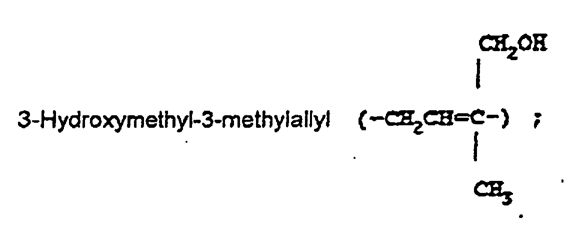 und wobei die Konzentration zwischen ungefähr 10 ppm und ungefähr 1000 ppm beträgt und ausreichend ist, um die Bindegewebsschicht des Gastrointestinaltrakts zu durchdringen und dadurch die nachteiligen Alterungseffekte auf die Zellen zu verringern.

## Revendications

1. Procédé pour atténuer les effets néfastes du vieillissement sur des cellules de mammifère en culture, comprenant la mise en contact des cellules de mammifère avec une composition qui contient une concentration efficace d'une cytokinine à noyau 6-(amino substitué)purine, dans lequel : la cytokinine à noyau 6-(amino substitué) purine répond à la formule I : 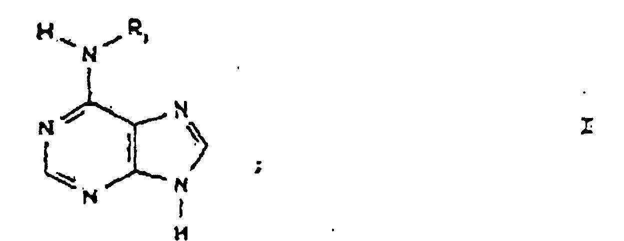 R₁ est choisi entre des groupes furfuryle, phényle, benzyle, n-alkyle ayant 4, 5 ou 6 atomes de carbone,
(cyclohexyl)méthyle (-CH₂(C₆H₁₁)),
et 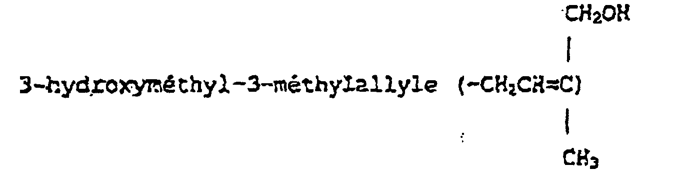 cytokinine qui est le seul agent actif présent à une concentration suffisante pour atténuer les effets néfastes du vieillissement des cellules, ce qui fait que la vitesse de développement des caractéristiques des cellules qui sont associées au vieillissement cellulaire est inversée ou ralentie ; et la vitesse de croissance et la capacité de prolifération totale des cellules après la mise en contact sont pratiquement identiques à celles existant avant cette mise en contact.

2. procédé suivant la revendication 1, dans lequel les cellules sont des fibroblastes.

3. Procédé suivant la revendication 1 ou la revendication 2, dans lequel la composition est un milieu de culture.

4. Procédé suivant l'une quelconque des revendications 1 à 3, dans lequel la concentration de la cytokinine à noyau 6-(amino substitué)purine est comprise dans l'intervalle de 0,5 ppm à 500 ppm.

5. Utilisation d'une composition qui contient une concentration comprise dans l'intervalle de 0,5 ppm à 500 ppm d'une cytokinine à noyau 6-(amino substitué)purine, dans lequel : la cytokinine à noyau 6-(amino substitué)purine répond à la formule I : 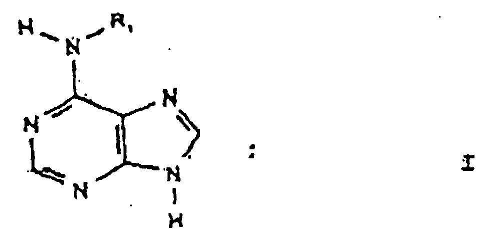 R₁ est choisi entre des groupes furfuryle, phényle, benzyle, n-alkyle ayant 4, 5 ou 6 atomes de carbone,
(cyclohexyl)méthyle (-CH₂(C₆H₁₁)),
et 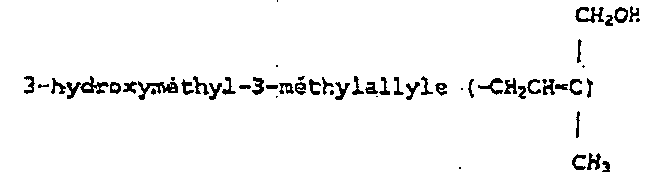 la gamme de concentrations étant suffisante pour atténuer les effets néfastes du vieillissement des cellules, ce qui fait que la vitesse de développement des caractéristiques des cellules qui sont associées au vieillissement cellulaire est inversée ou ralentie ; et la vitesse de croissance et la capacité de prolifération totale des cellules après la mise en contact sont pratiquement identiques à celles existant avant cette mise en contact, dans la production d'un médicament pour atténuer les effets néfastes du vieilissement sur des cellules cutanées diploïdes normales de mammifère.

6. Utilisation suivant la revendication 5, dans laquelle les cellules sont des fibroblastes cutanés.

7. Utilisation suivant la revendication 6, dans laquelle la composition est formulée à l'état de lotion, de pommade, de crème, de bâtonnet solide ou de liquide d'atomisation pour l'application à la surface de la peau.

8. Utilisation suivant l'une quelconque des revendications précédentes, dans laquelle le groupe R₁ dans la formule I est un groupe furfuryle ou 3-hydroxyméthyl-3-méthylallyle.

9. Utilisation ou procédé suivant l'une quelconque des revendications précédentes, dans lequel la concentration de la cytokinine à noyau 6-(amino substitué)purine est comprise dans l'intervalle de 10 ppm à 100 ppm.

10. Utilisation ou procédé suivant l'une quelconque des revendications précédentes, dans lequel la cytokinine à noyau 6-(amino substitué)purine est la kinétine.

11. Composition pour atténuer les effets néfastes du vieillissement sur des cellules de mammifère, comprenant une quantité comprise dans l'intervalle de 0,5 ppm à 500 ppm d'une cytokinine à noyau 6-(amino substitué)purine répondant à la formule I : 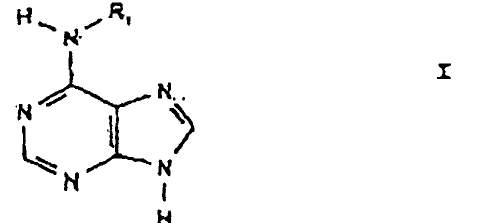 dans laquelle R₁ est choisi entre des groupes furfuryle, phényle, benzyle, n-alkyle ayant 4, 5 ou 6 atomes de carbone,
(cyclohexyl)méthyle (-CH₂(C₆H₁₁)),
et

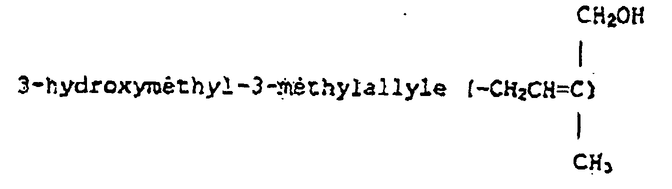

12. Composition suivant la revendication 11 qui est un produit cosmétique.

13. Composition suivant la revendication 12, qui est formulée pour une application topique à la surface de la peau d'un mammifère.

14. Composition cosmétique suivant la revendication 12 ou la revendication 13, dans laquelle la composition est formulée à l'état de lotion, de pommade, de crème, de bâtonnet solide ou de liquide d'atomisation.

15. Composition suivant l'une quelconque des revendications 11 à 14, dans laquelle le groupe R₁ de la formule I est un groupe furfuryle ou 3-hydroxyméthyl-3-méthylallyle.

16. Composition suivant l'une quelconque des revendications 11 à 14, dans laquelle la cytokinine à noyau 6-(amino substitué)purine de formule I est la kinétine.

17. Composition suivant l'une quelconque des revendications 11 à 16, dans laquelle la concentration du composé de formule I est comprise dans l'intervalle de 10 ppm à 100 ppm.

18. Composition orale pour atténuer les effets néfastes du vieillissement sur des cellules de mammifère, comprenant une concentration efficace d'une cytokinine à noyau 6-(amino substitué)purine dans un véhicule physiologiquement acceptable, dans laquelle :
la cytokinine à noyau 6-(amino substitué) purine répond à la formule I : 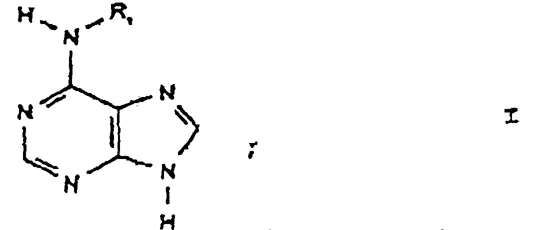 dans laquelle R₁ est choisi entre des groupes furfuryle, phényle, benzyle, n-alkyle ayant 4, 5 ou 6 atomes de carbone,
(cyclohexyl)méthyle (-CH₂(C₆H₁₁)),
et 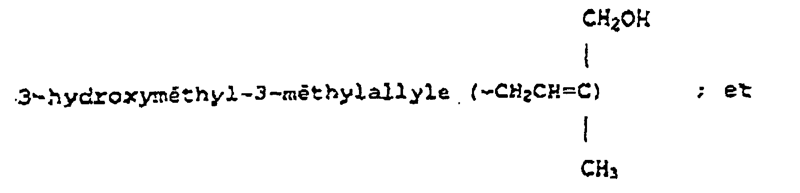 la concentration est comprise dans l'intervalle d'environ 10 ppm à environ 1000 ppm et est suffisante pour permettre de passer à travers la couche de tissu conjonctif du tractus gastro-intestinal et, ainsi, atténuer les effets néfastes du vieillissement sur les cellules.
